# EUROPÄISCHE PATENTANMELDUNG

(11) **EP 2 335 672 A2**
(43) Veröffentlichungstag der Anmeldung: **22.06.2011**
(21) Anmeldenummer: 10187729.8
(22) Anmeldetag: 15.10.2010
(51) Int. Cl.: A61K 8/04, A61K 8/31, A61K 8/37, A61K 8/81, A61Q 5/00, A61Q 5/12

(54) **Haarkur ohne Silikone**

(30) Priorität: 19.11.2009 DE 102009046869
(71) Anmelder: Henkel AG & Co. KGaA, 40589 Düsseldorf (DE)
(72) Erfinder: Battermann, Marlene, 21271, Asendorf (DE); Delowsky, Jens, 22844, Norderstedt (DE); Westphal, Petra, 21629, Neu Wulmstorf (DE); Hippe, Thomas, 25482, Appen (DE)

(57) **Zusammenfassung**

Die Erfindung betrifft Haarbehandlungsmittel enthaltend ein kationisches Copolymer und Esteröle bei gleichzeitiger Abwesenheit von Silikonen sowie die Verwendung dieser Mittel zur Haarbehandlung.

## Beschreibung

Die Erfindung betrifft Haarbehandlungsmittel enthaltend ein kationisches Copolymer und Esteröle bei gleichzeitiger Abwesenheit von Silikonen sowie die Verwendung dieser Mittel zur Haarbehandlung.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften. Insbesondere Silikonöle werden in haarkosmetischen Zusammensetzungen als Wirkstoffe eingearbeitet. Selbst bei sachgemäßer Anwendung dieser Produkte ist eine gute Produktleistung auf dem Haar nicht gewährleistet. Gesucht sind daher weiterhin stabile Systeme bei gleichzeitiger Abwesenheit von Silikonölen.

Überraschenderweise wurde nun jedoch gefunden, dass bestimmte kationische Polymere gemeinsam mit Esterölen zu stabilen wirksamen Zusammensetzungen führen.

Gleichzeitig führt die Verwendung dieser Kombinationen zu überraschend guten Eigenschaften des behandelten Haares, insbesondere zu verbesserten Kämmbarkeiten und zu einer verbesserten Elastizität als auch zu einer deutlich gesteigerten Waschbeständigkeit gefärbten Haares, sowie zu einer längeren Haltbarkeit bei einer gleichzeitigen besseren Umformleistung bei Wellvorgängen wie Wasserwelle und Dauerwelle. Insbesondere wird jedoch der Glanz des damit behandelten Haares deutlich erhöht.

Diese positiven Eigenschaften werden sowohl bei einer typischen rinse-off als auch bei einer leave-on Applikation erhalten. In hervorragender Weise eignet sich die erfindungsgemäße Zusammensetzung jedoch für die Sprühapplikation als non-Aerosol. Hierbei wird gegenüber den bislang üblichen Produkten ein außerordentlich gleichmäßiges Sprühbild erzielt. Dadurch wird die gesamte Zusammensetzung sehr gleichmäßig auf dem Haar verteilt.

Ein erster Gegenstand der vorliegenden Erfindung ist daher eine Zusammensetzung zur Behandlung von keratinischen Fasern, enthaltend
a) ein kationisches Copolymer bestehend aus einem Monomer der Formel (l) und einem Monomer der Formel (II) und
b) mindestens ein kosmetisches ÖI bei gleichzeitiger Abwesenheit von Silikonölen
   und
c) einen kosmetischen Träger.

Die Inhaltsstoffe a), b) und c) werden nachfolgend detailliert beschrieben. Soweit nachstehend vom Wirkstoffkomplex (A) gesprochen wird, bezieht sich diese Aussage auf die in den erfindungsgemäßen Mitteln zwingend enthaltenen Inhaltsstoffe a), b) und c).

Haarbehandlungsmittel im Sinne der vorliegenden Erfindung sind beispielsweise Haarkonditionierer, Haarsprays, Haarspülungen, Haarkuren, Haarpackungen, Haar-Tonics, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Shampoos, Konditioniermittel oder Haar-Tonics.

Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Messparameter können durch den Fachmann sensorisch beurteilt oder durch Messeinrichtungen quantifiziert werden.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Als kosmetische Träger c) eignen sich erfindungsgemäß besonders O/W - , W/O - und W/O/W - Emulsionen in Form von Cremes oder Gelen oder andere Zubereitungen, die insbesondere für die Anwendung auf dem Haar geeignet sind. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Als Inhaltstoff a) enthalten die erfindungsgemäßen Mittel mindestens ein kationisches Copolymer. Das kationische Copolymer ist hierbei aus den Monomeren der Formel (1) und (2) aufgebaut, 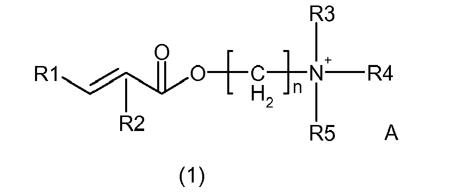 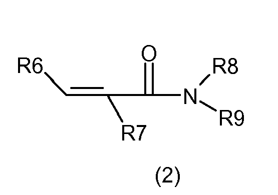 in welchen R1 bis R9 unabhängig voneinander Wasserstoff, C1 - C4 Alkyl, Methyl, Ethyl, Propyl, iso-Propyl, Butyl, iso-Butyl oder tert-Butyl bedeutet und der Maßgabe, dass mindestens einer der Reste R6, R7, R8 oder R9 C1 - C4alkyl bedeutet, n ist ganzzahlig von 1 bis 8 und A steht für ein physiologisch verträgliches Anion wie Halogenid, Fluorid, Chlorid, Bromid, Iodid, Hydrogensulfat oder Methosulfat. Vorzugsweise ist R1 Wasserstoff oder eine Methylgruppe, besonders bevorzugt ist R1 eine Methylgruppe. Vorzugsweise ist R2 Wasserstoff oder eine C1-C4alkylgruppe, besonders bevorzugt ist R2 Wasserstoff. Vorzugsweise ist R3, R4 und R5 unabhängig voneinander Wasserstoff oder eine C1-C4alkylgruppe, insbesondere eine Methylgruppe. Besonders bevorzugt ist R3, R4 und R5 gleich und höchst bevorzugt Methyl. Die Zahl n ist bevorzugt ganzzahlig von 1 bis 7, bevorzugter von 1 bis 5, höchst bevorzugt 1, 2, 3 oder 4 und am bevorzugtesten 2. R6 ist bevorzugt Wasserstoff oder Methyl. R7 ist bevorzugt Wasserstoff, Methyl oder Ethyl, besonders bevorzugt Methyl. R8 und R9 sind bevorzugt gleich und stehen für Wasserstoff, Methyl oder C1 - C4alkyl, besonders bevorzugt Wasserstoff oder Methyl. Am bevorzugtesten ist das Monomer der Formel (2) Dimethylacrylamid. Das erfindungsgemäße Copolymer besteht zu 20 bis 95 Gew.% aus dem Monomeren (1) und zu 5 bis 50 Gew.% aus dem Monomeren (2). Bevorzugt ist das Monomer (1) zu 40 bis 90 Gew.% und das Monomer (2) zu 10 bis 40 Gew.% enthalten.

Selbstverständlich kann das erfindungsgemäße kationische Copolymer weiterhin vernetzt sein. In diesem Falle wird die Vernetzung durch die üblichen Vernetzungsmittel wie beispielsweise Allylacrylamid, Allylmethacrylamid, Tetraallylammoniumchlorid oder N,N -methylen-bisacrylamid in Mengen bis zu 500 ppm bewirkt.

Weiterhin ist erfindungsgemäß umfasst, dass das kationische Copolymer als eine Lösung oder Suspension oder Dispersion in geeigneten kosmetischen Medien, wie beispielsweise Propylenglykol, Glycerin, Paraffin, Isoparaffin, Propylenglykolen und Estern von Propylenglykol wie beispielsweise Propylenglykol Dicaprylate/Dicaprate, oder PPG-1 Trideceth-6 und weiteren vorliegen kann. Erfindungsgemäß am bevorzugtesten ist das Handelsprodukt Tinovis^{®} CD der Firma Ciba.

Das erfindungsgemäße kationische Copolymer wird in einer Menge von 0,1 bis 10 Gew.% bezogen auf die gesamte Zusammensetzung verwendet. Bevorzugt sind Mengen von 0,1 bis 5 Gew.%, besonders bevorzugt von 0,1 bis 3,0 Gew.%.

Die vorliegende erfindungsgemäße Wirkstoffkombination kann als rinse-off Anwendung oder als leave-on Anwendung konfektioniert werden. Eine Anwendung als leave-on Anwendung ist bevorzugt. Innerhalb der leave-on Anwendungen ist eine Konfektionierung als Non-Aerosol Spray besonders bevorzugt.

Der Inhaltsstoff b) der erfindungsgemäßen Zusammensetzungen ist ein kosmetisches ÖI. Bevorzugt weisen diese Ölkörper einen Schmelzpunkt kleiner als 50°C, besonders bevorzugt kleiner als 45 °C, ganz besonders bevorzugt kleiner als 40°C, höchst bevorzugt kleiner als 35 °C und am bevorzugtesten sind die kosmetischen Öle bei einer Temperatur kleiner als 30°C fließfähig. Im Folgenden werden diese Öle näher definiert und beschrieben.

Zu den natürlichen und synthetischen kosmetischen Ölen sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 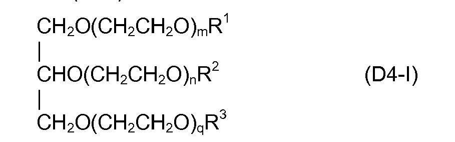 in der R¹ R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnussöl oder Wildrosenöl.

Die bevorzugtesten kosmetischen Öle sind
b1) flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen,
b2) Esteröle und
b3) symmetrische, unsymmetrische oder cyclische Ester der Kohlensäure.

Bevorzugt ist die Anwesenheit von mindestens 2 unterschiedlichen Ölen der kosmetischen Öle b1 bis b3). Hierbei ist es vorteilhaft, wenn ein ÖI der Gruppe b2 mit einem ÖI der Gruppe b3 gemeinsam verwendet wird. Am bevorzugtesten jedoch sind Zusammensetzungen, welche alle drei Gruppen b1) bis b3) enthalten.

Die kosmetischen Öle sind in den erfindungsgemäßen Zusammensetzungen in Mengen von 0,1 bis 20 Gew.% enthalten.

In vielen Fällen enthalten die Mittel mindestens eine oberflächenaktive Substanz, wobei prinzipiell sowohl anionische als auch zwitterionische, ampholytische, nichtionische und kationische oberflächenaktive Substanzen geeignet sind. Die Auswahl der oberflächenaktiven Substanzen richtet sich nach der Art des Mittels. Im Falle eines Shampoos wird insbesondere mindestens ein Tensid aus der Gruppe der anionischen, der zwitterionischen oder nichtionischen oberflächenaktiven Substanzen gewählt. Bevorzugt ist hierbei, dass mindestens eine anionische und mindestens eine zwitterionische oberflächenaktive Substanz gewählt wird. Besonders bevorzugt werden diese oberflächenaktiven Substanzen dabei aus der Gruppe der besonders milden oberflächenaktiven Substanzen gewählt. In vielen Fällen hat es sich aber als vorteilhaft erwiesen, die Tenside aus anionischen, zwitterionischen oder nichtionischen Tensiden auszuwählen. Dabei beträgt das Verhältnis zwischen anionischen und zwitterionischen oberflächenaktiven Substanzen zwischen 10 : 1 und 1 : 5. Besonders bevorzugt ist das Verhältnis 5 : 1 bis 1 : 2.

Als anionische Tenside (Tanion) eignen sich in erfindungsgemäßen Zubereitungen alle für die Verwendung am menschlichen Körper geeigneten anionischen oberflächenaktiven Stoffe. Folgende anionische Tenside haben sich als mild bis besonders mild erwiesen und sind erfindungsgemäß besonders bevorzugt:
- Acyllactylate,
- Hydroxymischethersulfate,
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist und deren Salze,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen.
- Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, R¹(OCH₂CH₂)ₙ-O-(PO-OX)-OR²,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und Xfür Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- Monoglyceridsulfate und Monoglyceridethersulfate der Formel ROC-(OCH₂CH₂kOCHᵣ[CHO(CH₂CH₂0 )yH]-CH₂0(CH₂CH₂0)_{z}-S0₃X,
   in der RCO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der RCO für einen linearen Acylrest mit 8 bis 18 Kohlenstoffatomen steht,
- Amidethercarbonsäuren, R¹-CO-NR²-CH₂CH₂O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo^{®} erhältlich.
- Kondensationsprodukte aus einem wasserlöslichen Salz eines wasserlöslichen Eiweißhydrolysates mit einem geeigneten Fettsäurederivat, beispielsweise einem C8 - C30 Fettsäurehalogenid. Solche Produkte sind unter dem Warenzeichen Lamepon^{®}, Maypon^{®}, Gluadin^{®}, Hostapon^{®} KCG oder Amisoft^{®} seit langem im Handel erhältlich,
- Acylglutamate und
- Acylaspartate.

Weiterhin ist es im Falle von milden anionischen Tensiden mit Polyglycolethereinheiten bevorzugt, dass die Zahl der Glykolethergruppen 1 bis 20 beträgt, bevorzugt 2 bis 15, besonders bevorzugt 2 bis 12. Besonders milde anionische Tenside mit Polyglykolethergruppen ohne eingeschränkte Homologenverteilung können beispielsweise auch erhalten werden, wenn einerseits die Zahl der Polyglykolethergruppen 4 bis 12 beträgt und als Gegenion Zn- oder Mg-ionen gewählt werden. Beispiel hierfür ist das Handelsprodukt Texapon^{®} ASV.

Als zwitterionische Tenside (Tzwitter) werden solche oberflächenaktiven Verbindungen bezeichnet, die im Molekül mindestens eine quartäre Ammoniumgruppe und mindestens eine -COO⁽⁻⁾ - oder - S0₃(-) -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin. Nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.
- Zuckertenside vom Typ der Fettsäure-N-alkylpolyhydroxyalkylamide, ein nichtionisches Tensid der Formel (Tnio-3),

   R⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Eine ganz besonders bevorzugte Ausführungsform enthält mindestens ein kationisches Tensid. Kationische Tenside (Tkat) leiten sich im allgemeinen von Ammoniumionen ab und besitzen eine Struktur

(NR¹R²R³R⁴)⁺A⁻ (Tkat1)

mit einem entsprechend negativ geladenen Gegenion A.

Bei allen erfindungsgemäßen kationischen Tensiden ist das Anion A ausgewählt aus den physiologisch verträglichen Anionen. Beispielhaft für das Anion seien die Halogenide, Fluorid, Chlorid, Bromid, Iodid, Phosphat, Dihydrogenphosphat, Hydogenphosphat, Hydrogensulfat, Sulfat (Methosulfat) der allgemeinen Formel RSO₃ worin R die Bedeutung von gesättigtem oder ungesättigtem Alkylresten mit 1 bis 4 Kohlenstoffatomen hat, insbesondere Methosulfat und Ethosulfat, oder anionische Reste organischer Säuren wie Maleat, Fumarat, Oxalat, Tartrat, Citrat, Lactat oder Acetat, genannt. Besonders bevorzugte kationische Tenside haben als Anion das Chlorid oder Methosulfat mit einer Methyl-, oder Ethylgruppe als Rest R.

Derartige kationische Ammoniumverbindungen der Formel (Tkat1) sind dem Fachmann bestens bekannt. Die Reste R1 bis R4 können jeweils unabhängig voneinander geradkettige, verzweigte, cyclische, aromatische gesättigte oder ungesättigte Alkyl- und/oder Alkenylketten mit mindestens 1 bis 30 Kohlenstoffatomen, Wasserstoff, -OH oder Hydroxyethyl- bedeuten.

Erfindungsgemäß bevorzugt sind kationische Strukturen, in welchen die Reste R1,R2 und R3 jeweils eine Methylgruppe bedeuten und R4 einen gesättigten, verzweigten oder unverzweigten Alkylrest mit einer Kettenlänge von 16 bis 24 Kohlenstoffatomen darstellt und weiterhin der Rest R4 einen gesättigten, verzweigten oder unverzweigten Alkylrest, ganz besonders bevorzugt unverzweigten Alkylrest mit einer Kettenlänge von 18 bis 24, höchst bevorzugt mit einer Kettenlänge von 22 bis 24 Kohlenstoffatomen darstellt. Bevorzugte quaternäre Ammoniumverbindungen der Struktur (Tkat1) sind Ammoniumhalogenide, insbesondere Chloride und Bromide, wie Alkyltrimethylammoniumchloride, Dialkyldimethylammoniumchloride und Trialkylmethylammoniumchloride, z.B. Distearyldimethylammoniumchlorid, Lauryldimethylammoniumchlorid, Lauryldimethylbenzylammoniumchlorid und Tricetylmethylammoniumchlorid Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze. Besonders bevorzugt sind letztere in Form der Methosulfate und der Bromide. Am bevorzugtesten sind Cetyltrimethylammoniummethosulfat und Behenyltrimethylammoniummethosulfat und höchst bevorzugt ist Behenyltrimethylammoniummethosulfat.

Weiterhin können in einer Ausführungsform Esterquats gemäß der Formel (Tkat1-2) verwendet werden.

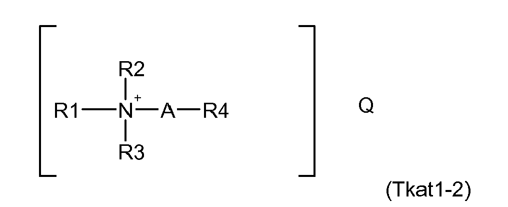

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, beispielsweise Methyl, Ethyl, Propyl, Isopropyl, Butyl, Isobutyl, t-Butyl, Hydroxyethyl, Hydroxymethyl, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, dass höchstens 2 der Reste R1, R2 oder R3 für diesen Rest stehen können:
   Der Rest -(A - R4) ist mindestens 1 bis 3 mal enthalten.

Hierin steht A für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
und Q steht für ein physiologisch verträgliches organisches oder anorganisches Anion, welches wie zuvor unter Formel (Tkat1) beschrieben wurde. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Rewoquat^{®}, Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG und Stepantex^{®} GS 90 sind Beispiele für solche Esterquats.

Weitere erfindungsgemäß besonders bevorzugte Verbindungen der Formel (Tkat1-2) zählen zur Formel (Tkat1-2.1), den kationischen Betainestern. 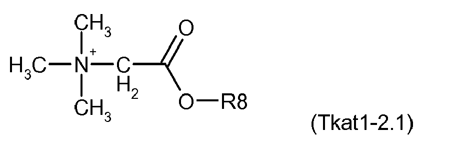 R8 entspricht in seiner Bedeutung R7.

Weitere bevorzugte kationische Tenside sind kationische Tenside der Formel (Tkat-2). 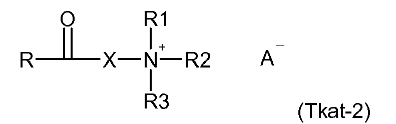 R steht hierin für einen substituierten oder unsubstituierten, verzweigten oder geradkettigen Alkyl- oder Alkenylrest mit 11 bis 35 Kohlenstoffatomen in der Kette,
X steht für-O-oder-NR⁵-,
R¹ steht für eine Alkylengruppe mit 2 bis 6 C - Atomen, welche nicht substituiert oder substituiert sein kann, wobei im Falle einer Substitution die Substitution mit einer -OH - oder -NH- Gruppe bevorzugt ist,
R² und R³ jeweils unabhängig voneinander stehen für eine Alkyl oder Hydroxyalkylgruppe mit 1 bis zu 6 C - Atomen in der Kette, wobei die Kette geradlinig oder verzweigt sein kann. Beispiele für erfindungsgemäße Reste sind Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyalkyl, Dihydroxyalkyl, Hydroxyethyl, Hydroxypropyl, Dihydroxypropyl, Hydroxybutyl, Dihydroxybutyl, Trihydroxybutyl, Trihydroxypropyl, Dihydroxyethyl,
R5 steht für Wasserstoff oder einen C1 bis C6 geradkettigen oder verzweigten, Alkyl- oder Alkenylrest, welcher auch durch eine Hydroxygruppe substituiert sein kann, besonders Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyethyl, Hydroxypropyl, Dihydroxypropyl, Hydroxybutyl, Dihydroxybutyl, Trihydroxybutyl, Trihydroxypropyl, Dihydroxyethyl und
A⁻ bedeutet ein Anion wie bereits bei der Formel (Tkat1) beschrieben.

Innerhalb dieser Strukturklasse werden bevorzugt die Verbindungen einer der folgenden Strukturen verwendet:

CH₃(CH₂)₂₀CONH(CH₂)₃ - N⁺(CH₃)₂-CH₂CH₃ A⁻ (Tkat-3)

CH₃(CH₂₀CONH(CH₂)₃ - N⁺(CH₃)₂-CH₂(CHOH)CH₂OH A⁻ (Tkat-4)

CH₃(CH2)₂₀COOCH₂CHOHCH₂ - N⁺(CH₃)₃ A⁻ (Tkat-5)

CH₃(CH₂)₂₀CONH(CH₂)₃ - N⁺(CH₃)₂-CH₂CH₂OH A⁻ (Tkat-6)

Beispiele für derartige Handelsprodukte sind Schercoquat BAS, Lexquat AMG-BEO, Akypoquat 131 oder Incroquat Behenyl HE.

In einer besonders bevorzugten Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit den folgenden Strukturformeln:

R¹ - NH - (CH₂)ₙ - NR²R³ (Tkat7)

und/oder

R¹ - NH - (CH₂)ₙ - NR ²R ³R⁴ (Tkat8)

worin R1 ein Acyl- oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander Wasserstoff oder ein Alkylrest mit 1 bis 4 C-Atomen, welcher gleich oder verschieden, gesättigt oder ungesättigt sein kann, und
n eine ganze Zahl zwischen 1 und 10 bedeuten.

Das Anion ist wiederum ausgewählt aus den physiologisch verträglichen Anionen wie bereits bei der Formel (Tkat1) beschreiben.

Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen-oder Candellilawachse in Betracht. Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5.

Weiterhin bevorzugt sind Amidoamine und/oder quaternisierte Amidoamine der allgemeinen Formeln (Tkat7) und/oder (Tkat8), in denen das Anion ein Halogenidion oder eine Verbindung der allgemeinen Formel RSO₃ ist, worin R die Bedeutung von gesättigtem oder ungesättigtem Alkylresten mit 1 bis 4 Kohlenstoffatomen hat.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Als erfindungsgemäß zu verwendende Amidoamine, welche gegebenenfalls quaternisiert sein können, kommen beispielsweise in Betracht als Amidoamine: Witcamine 100 (Witco, INCI-Bezeichnung: Cocamidopropyl Dimethylamine), Incromine BB (Croda, INCI-Bezeichnung: Behenamidopropyl Dimethylamine), Mackine 401 (Mclntyre, INCI-Bezeichnung: Isostearylamidopropyl Dimethylamine) und andere Mackine-Typen, Adogen S18V (Witco, INCI-Bezeichnung: Stearylamidopropyl Dimethylamine), und als permanent kationische Aminoamine: Rewoquat RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quatemium-33), Rewoquat UTM 50 (Witco Surfactants GmbH, Undecyleneamidopropyltrimonium Methosulfate).

Schließlich können als kationische Tenside quartäre Imidazolinverbindungen, d.h. Verbindungen, die einen positiv geladenen Imidazolinring aufweisen, verwendet werden. Die im Folgenden dargestellte Formel (Tkat9) zeigt die Struktur dieser Verbindungen.

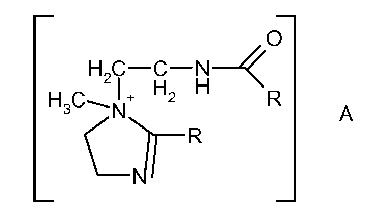

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat9) enthalten für R jeweils den gleichen Kohlenwasserstoffrest. Die Reste R stehen unabhängig voneinander jeweils für einen gesättigten oder ungesättigten, linearen oder verzweigten Kohlenwasserstoffrest mit einer Kettenlänge von 8 bis 18 Kohlenstoffatomen. Die bevorzugten Verbindungen der Formel enthalten für R jeweils den gleichen Kohlenwasserstoffrest. Die Kettenlänge der Reste R beträgt bevorzugt 12 bis 18 Kohlenstoffatome. Besonders bevorzugt sind Verbindungen mit einer Kettenlänge von mindestens 16 Kohlenstoffatomen und ganz besonders bevorzugt mit 18 Kohlenstoffatomen. Die Kettenlänge der Reste R beträgt höchst bevorzugt mindestens 20 Kohlenstoffatome. Am bevorzugtesten sind Verbindungen mit einer Kettenlänge von mindestens 21 Kohlenstoffatomen. Bevorzugte Reste R sind Cetyl, Oleyl, Palmityl, Stearyl und Behenyl. A bedeutet ein physiologisch verträgliches Anion, wie bereits bei den kationischen Verbindungen der Formel (Tkat1) beschrieben. Besonders erfindungsgemäße Beispiele sind unter den INCII - Bezeichnungen Quaternium-27, Quaternium-72 und Quaternium-83 erhältlich. Ein höchst bevorzugtes Handelsprodukt dieser Kettenlänge ist beispielsweise unter der Bezeichnung Quaternium-91 bekannt.

Selbstverständlich umfasst die erfindungsgemäße Lehre auch, dass mindestens zwei kationische Tenside verwendet werden. In diesem Falle ist es bevorzugt, wenn die kationischen Tenside aus zwei unterschiedlichen Strukturklassen gewählt werden.

Die kationischen Tenside sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Kationische, zwitterionische und/oder amphotere Tenside sowie deren Mischungen können erfindungsgemäß bevorzugt sein.

Die Tenside (T) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate wie bereits bei den Tensiden beschrieben,
- Monoglyceridsulfate und Monoglyceridethersulfate wie bereits bei den Tensiden beschrieben,
- Amidethercarbonsäuren wie sie im Kapitel über Tenside beschrieben sind,
- Kondensationsprodukte aus einem wasserlöslichen Salz eines wasserlöslichen Eiweißhydrolysat-Fettsäure-Kondensationsproduktes,
- Zwitterionische Tenside (Tzwitter)),
- Ampholytische Tenside (Tampho),
- Zuckertenside vom Typ der Alkyl- oder Alkenyloligoglykoside,
- Zuckertenside vom Typ der Fettsäure-N-alkylpolyhydroxyalkylamide,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- Aminoxide,
- Hydroxymischether, beispielsweise der Formel R¹O[CH₂CH(CH₃)O]_{X}(CH₂CHR²O)_{Y}[CH₂CH(OH)R³]_{Z} mit R¹ stehend für einen linearen oder verzweigten, gesättigten oder ungesättigten Alkyl- und/oder Alkenylrest mit 2 bis 30 C - Atomen, R² stehend für Wasserstoff, einen Methyl-, Ethyl-, Propyl- oder iso-Propylrest, R³ stehend für einen linearen oder verzweigten Alkylrest mit 2 bis 30 C - Atomen, x stehend für 0 oder eine Zahl von 1 bis 20, Y für eine Zahl von 1 bis 30 und z stehend für die Zahl 1, 2, 3 , 4 oder 5.
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),
- Esterquats,
- Alkylamidoamine und quaternierte Alkylamidoamine.
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine,
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),

In den erfindungsgemäßen Mitteln werden ganz besonders kationische und/oder amphotere und/oder zwitterionische Polymere als weitere Inhaltsstoffe verwendet.

Im Folgenden werden einige Beispiele von besonders bevorzugten Polymeren beschrieben.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacrylamidopropyltrimethylammoniumchlorid Copolymer.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich. Weitere Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen.

Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®}vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydageno^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationiserter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt.

Die kationischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Amphotere Polymere sind ebenso wie die kationischen Polymere ganz besonders bevorzugte Polymere. Besonders bevorzugte amphotere Polymere sind Copolymere, aus Diallyldimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben. Erfindungsgemäß ganz besonders bevorzugt verwendete amphotere Polymere sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Die amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt ist das Homopolymer der 2-Acrylamido-2-methylpropansulfon-säure, das beispielsweise unter der Bezeichnung Rheothik^{®}11-80 im Handel erhältlich ist.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaflin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die nichtionischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Die Polymere (P) sind in den erfindungsgemäß verwendeten Zusammensetzungen bevorzugt in Mengen von 0,01 bis 30 Gew.-%, bezogen auf die gesamte Zusammensetzung, enthalten. Mengen von 0,01 bis 25, insbesondere von 0,01 bis 15 Gew.-%, sind besonders bevorzugt.

Mit besonderem Vorzug enthalten die erfindungsgemäßen Zusammensetzungen Fettstoffe (Fat) als weiteren Wirkstoff. Unter Fettstoffen (Fat) sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®}871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole können ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} Ooder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 -20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an ÖI- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Ein weiterer erfindungsgemäßer synergistischer Wirkstoff in den erfindungsgemäßen Zusammensetzungen mit dem erfindungsgemäßen Wirkstoffkomplex sind Proteinhydrolysate und/oder dessen Derivate (Prot).

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Am bevorzugtesten sind Proteinhydrolysate auf der Basis von Kollagen, Seide, Keratin, Milcheiweiß, Soja, Mandeln, Weizen, Fischen, Algen oder Perlen. Höchst bevorzugt sind die Hydrolysate auf der Basis von Seide, Keratin, Milcheiweiß, Fischen, Algen oder Perlen.

Die Proteinhydrolysate (Prot) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 -5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Schließlich ergeben sich durch die Verwendung von Pflanzenextrakten (L) in den erfindungsgemäßen Zusammensetzungen weitere Vorteile. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa und Ingwerwurzel bevorzugt. Erfindungsgemäß kann es höchst bevorzugt sein, wenn als Pflanzenextrakte sogenannte ayurvedische Pflanzenextrakte verwendet werden. Zu den traditionellen ayurvedischen Pflanzen zählen Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala).

Als weiteren wesentlichen Inhaltsstoff können die erfindungsgemäßen Mittel Purin und/oder Derivat(e) des Purins enthalten. Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß folgende Verbindungen bevorzugt: Purin, Adenin, Guanin, Harnsäure, Hypoxanthin, 6-Purinthiol, 6-Thioguanin, Xanthin, Coffein, Theobromin und Theophyllin. In haarkosmetischen Formulierungen hat sich insbesondere Coffein bewährt, das beispielsweise in Shampoos, Conditionern, Haarwässern und/oder Lotionen vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf die Zusammensetzung) eingesetzt werden kann.

Ein weiterer bevorzugter Wirkstoff zur zusätzlichen Verwendung in den erfindungsgemäßen Mitteln ist Taurin und/oder ein Derivat des Taurines. Unter Taurin wird ausschließlich 2-Aminoethansulfonsäure und sowie explizit genannte Derivate des Taurines verstanden. Unter den Derivaten des Taurines werden N-Monomethyltaurin und N,N-Dimethyltaurin verstanden. Als weitere Taurinderivate werden auch Taurine verstanden, welche als Stoffwechselprodukte im pflanzlichen und/oder tierischen und/oder marinen Organismen natürlicherweise vorkommen. Hierzu zählen beispielsweise, wenn auch nicht bevorzugt, Abbauprodukte des Cysteines, insbesondere die Cysteinsulfinsäure. Weitere Taurinderivate im Sinne der vorliegenden Erfindung sind die Taurocholsäure und Hypotaurin.

Besonders bevorzugte sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. Zusätzlich kann es sich als vorteilhaft erweisen, wenn in den erfindungsgemäßen Zusammensetzungen Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Eine besonders bevorzugte Gruppe von Quellmitteln können Hydantoine sein. Erfindungsgemäße Zusammensetzungen enthalten bevorzugt 0,01 bis 5 Gew.-% Hydantoin bzw. mindestens eines Hydatoinderivates. Besonders bevorzugt werden erfindungsgemäß Hydantoinderivate eingesetzt, wobei 5-Ureidohydantoin besonders bevorzugt ist. Unabhängig davon, ob Hydantoin oder Hydantoinderivat(e) eingesetzt wird/werden, sind Einsatzmengen von 0,02 bis 2,5 Gew.-% ganz besonders bevorzugt, von 0,05 bis 1,5 Gew.-%, weiter bevorzugt 0,075 bis 1 Gew.-% und insbesondere 0,1 bis 0,25 Gew.-% -jeweils bezogen auf das gesamte Mittel - höchst bevorzugt. Die erfindungsgemäße Verwendung von Polyhydroxyverbindungen als Wirkstoff mit den anderen erfindungsgemäßen Komponenten kann besonders bevorzugt sein. Unter Polyhydroxyverbindungen sind organische Verbindungen mit mindestens zwei Hydroxygruppen zu verstehen. Insbesondere sind im Sinne der vorliegenden Erfindung hierunter zu verstehen:
- Polyole mit mindestens zwei Hydroxygruppen, wie beispielsweise Trimethylolpropan,
- Kohlenhydrate, Zuckeralkohole und Zucker sowie deren Salze,
- insbesondere Monosaccharide, Disaccharide, Trisaccharide und Oligosaccharide, wobei diese auch in Form von Aldosen, Ketosen und/oder Lactosen vorliegen können,
- Aminodesoxyzucker, Desoxyzucker, Thiozucker, wobei diese auch in Form von Aldosen, Ketosen und/oder Lactosen vorliegen können,

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen vorliegen können.

Weiterhin sind bevorzugt Oligosaccharide mit bis zu 50 Monomereinheiten. Beispielhaft für die erfindungsgemäßen Polyole seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose.

Bevorzugte Polyhydroxyverbindungen sind Sorbit, Inosit, Mannit, Threit, Erythreit, Erythrose, Threose, Arabinose, Ribose, Xylose, Glucose, Galactose, Mannose, Allose, Fructose, Sorbose, Desoxyribose, Glucosamin, Galaktosamin, Saccharose, Lactose, Trehalose, Maltose und Cellobiose. Besonders bevorzugt werden Glucose, Galactose, Mannose, Fructose, Desoxyribose, Glucosamin, Saccharose, Lactose, Maltose und Cellobiose verwendet. Ganz besonders bevorzugt ist jedoch die Verwendung von Glucose, Galactose, Mannose, Fructose, Saccharose, Lactose, Maltose oder Cellobiose

Unter den Polyhydroxyverbindungen mit 3 OH-Gruppen hat das Glycerin eine herausragende Bedeutung.

Unabhängig vom Typ der eingesetzten Polyhydroxyverbindung mit mindestens 2 OH-Gruppen sind erfindungsgemäße Mittel bevorzugt, die, bezogen auf das Gewicht des Mittels, 0,01 bis 5 Gew.%, vorzugsweise 0,05 bis 4 Gew.%, besonders bevorzugt 0,05 bis 3,5 Gew.% und insbesondere 0,1 bis 2,5 Gew.% Polyhydroxyverbindung(en) enthalten.

Ganz besonders bevorzugte Polyole der vorliegenden Erfindung sind Polyole mit 2 bis 12 C-Atomen im Molekülgerüst. Diese Polyole können geradkettig, verzweigt, cyclisch und/oder ungesättigt sein. Die Hydroxygruppen sind dabei ganz besonders bevorzugt endständig benachbart oder endständig durch den Rest der Kette voneinander getrennt. Als Beispiele für diese Polyole seien genannt: Glykol, Polyethylenglykol bis zu einem Molgewicht bis zu 1000 Dalton, Neopentylglykol, Partialglycerinether mit einem Molgewicht bis zu 1000 Dalton, 1,2-Propandiol, 1,3-Propandiol, Glycerin, 1,2-Butandiol, 1,3-Butandiol, 1,4-Butandiol, 1,2,3-Butantriol, 1,2,4-Butantriol, Pentandiole, beispielsweise 1,2-Pentandiol, 1,5-Pentandiol, Hexandiole, 1,2-Hexandiol, 1,6-Hexandiol, 1,2,6-Hexantriol, 1,4-cyclo-Hexandiol, 1,2-cyclo-Hexandiol, Heptandiole, 1,2-Heptandiol, 1,7-Heptandiol, Oktandiole, 1,2-Oktandiol, 1,8-Oktandiol, 2-Ethyl-1,3-hexandiol, Octadienole, Decadienole, Dodekandiole, 1,2-Dodekandiol, 1,12-Dodekandiol, 1,12-Dodekandiol mit 10 Mol EO, Dodecadienole.

Die erfindungsgemäßen Polyhydroxyverbindungen sind in den Zusammensetzungen in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten.

In einer weiteren Ausführungsform sollten die erfindungsgemäßen Mittel zusätzlich mindestens einen UV-Lichtschutzfilter enthalten. Unter UV-Lichtschutzfiltern sind organische Substanzen zu verstehen, die in der Lage sind, ultra-violette Strahlen zu absorbieren und die aufgenommene Energie in Form längerwelliger Strahlung, z.B. Wärme wieder abzugeben. UVB-Filter können öllöslich oder wasserlöslich sein.

Ferner können die kosmetischen Mittel weitere Wirk-, Hilfs- und Zusatzstoffe enthalten, wie beispielsweise
- Strukturanten wie Maleinsäure und Milchsäure,
- Parfümöle, Dimethylisosorbid und Cyclodextrine,
- Farbstoffe zum Anfärben des Mittels,
- Antischuppenwirkstoffe wie Piroctone Olamine, Zink Omadine und Climbazol,
- Cholesterin,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher verwiesen.

Wie bereits erwähnt, kommt der hohen Pflegewirkung der erfindungsgemäßen Mittel insbesondere daher Bedeutung zu, als sie auch in Gegenwart von Oxidationsmitteln - beispielsweise im Rahmen der oxidativen Haarfärbung - hervorragende Ergebnisse liefert.

Ein zweiter Erfindungsgegenstand ist daher ein Verfahren zur Haarbehandlung, in dem ein kosmetisches Mittel gemäß Anspruch 1 auf das Haar aufgetragen wird und nach einer Einwirkungszeit vom Haar gespült wird.

Die Einwirkungszeit beträgt bevorzugt wenige Sekunden bis 100 Minuten, besonders bevorzugt 1 bis 50 Minuten und ganz besonders bevorzugt 1 bis 30 Minuten.

Die folgenden Beispiele sollen den Gegenstand der vorliegenden Erfindung erläutern ohne ihn jedoch zu beschränken.

Ein dritter Erfindungsgegenstand ist ein Verfahren zur Haarbehandlung, in dem ein kosmetisches Mittel gemäß Anspruch 1 auf das Haar aufgetragen wird und auf dem Haar bis zur nächsten Haarwäsche dort verbleibt.

Ein vierter Erfindungsgegenstand ist, dass die kosmetische Zusammensetzung als Non-Aerosol Sprühkur vorliegt und auf dem Haar aufgetragen wird und dort bis zur nächsten Haarwäsche verbleibt.

### Beispiele

Alle Mengenangaben sind, soweit nicht anders vermerkt, Gewichtsteile.

**Non Aerosol Sprayconditioner**

| | |
|---|---|
| D- Panthenol (75%ig) | 0,2 |
| Milchsäure (80%ig) | 0,35 |
| Methylparaben- Natriumsalz | 0,3 |
| Phenoxyethanol | 0,4 |
| Cetiol^{®} OE | 0,2 |
| Cetiol^{®} CC | 10,0 |
| Cetiol^{®} S | 0,5 |
| Dehyquart^{®} L80 | 1,3 |
| Tinovis^{®} CD | 1,7 |
| Parfüm | 0,35 |
| Wasser, demineralisiert | ad 100 |

## Patentansprüche

1. Haarkonditionierendes Mittel, enthaltend
a) ein kationisches Copolymer bestehend aus einem Monomer der Formel (I) und einem Monomer der Formel (II) und
b) mindestens ein kosmetisches Öl bei gleichzeitiger Abwesenheit von Silikonölen,
und
c) einen kosmetischen Träger.

2. Mittel nach Anspruch 1, **dadurch gekennzeichnet, dass** das kosmetische ÖI ausgewählt ist aus der Gruppe b1) den flüssigen Paraffinölen, Isoparaffinölen und synthetische Kohlenwasserstoffen sowie den Di-n-alkylethern mit insgesamt zwischen 12 bis 36 C-Atomen, insbesondere 12 bis 24 C-Atomen.

3. Mittel nach Anspruch 1, **dadurch gekennzeichnet, dass** das kosmetische ÖI ausgewählt ist aus der Gruppe b2) den Esterölen.

4. Mittel nach Anspruch 1, **dadurch gekennzeichnet, dass** das kosmetische ÖI ausgewählt ist aus der Gruppe b3) den symmetrischen, unsymmetrischen oder cyclischen Estern der Kohlensäure.

5. Mittel nach einem der Ansprüche 1 bis 4, **dadurch gekennzeichnet, dass** mindestens zwei unterschiedliche Öle aus den Gruppen der kosmetische Öle b1 bis b3 enthalten sind.

6. Mittel nach einem der Ansprüche 1 bis 5, **dadurch gekennzeichnet, dass** es zusätzlich mindestens einen Stoff aus der Gruppe der Vitamine, Provitamine und Vitaminvorstufen sowie deren Derivate enthält, wobei Vitamine, Pro-Vitamine und Vitaminvorstufen bevorzugt sind, die den Gruppen A, B, C, E, F und H zugeordnet werden.

7. Mittel nach einem der Ansprüche 1 bis 5, **dadurch gekennzeichnet, dass** es zusätzlich mindestens ein Proteinhydrolysat, insbesondere auf der Basis von Kollagen, Seide, Keratin, Milcheiweiß, Soja, Mandeln, Weizen, Fischen, Algen oder Perlen enthält.

8. Verwendung einer Zubereitung nach einem der Ansprüche 1 bis 6 zur Konditionierung von Haar.

9. Verfahren zur Behandlung von Haar, bei dem eine Zubereitung gemäß einem der Ansprüche 1 bis 3 auf das Haar aufgetragen wird und dort bis zur nächsten Haarwäsche verbleibt.
